# EUROPEAN PATENT SPECIFICATION

(11) **EP 2 430 444 B1**
(45) Date of publication and mention of the grant of the patent: **08.01.2014**
(21) Application number: 10721437.1
(22) Date of filing: 11.05.2010
(51) Int. Cl.: G01N 33/50, C12Q 1/66, G01N 33/58

(54) **METHOD FOR IDENTIFYING INHIBITORS AGAINST DENGUE VIRUS**
VERFAHREN ZUR IDENTIFIZIERUNG VON HEMMERN GEGEN DAS DENGUE-VIRUS
PROCÉDÉ POUR IDENTIFIER DES INHIBITEURS CONTRE LE VIRUS DE LA DENGUE

(30) Priority: 12.05.2009 EP 09159994
(43) Date of publication of application: 21.03.2012
(73) Proprietor: Janssen R&D Ireland, Eastgate, Little Island, County Cork (IE)
(72) Inventor: GONG, Edwin Yunhao, B-2340 Beerse (BE); IVENS, Tania Pauline Eduard, B-9120 Melsele (BE); CLYNHENS, Marleen, B-2830 Blaasveld (BE); LORY, Pedro Miguel Jales, B-2340 Beerse (BE); KRAUS, Guenter, B-2860 Sint-Katelijne-Waver (BE)
(74) Representative: van Wanrooij, Eva
(86) International application number: PCT/EP2010/056483
(87) International publication number: WO 2010/130748

(56) References cited:
- WO-A-00/18953
- WO-A-2005/010143
- WO-A-2006/085972
- WO-A-2009/016831
- WO-A1-02/095075
- Anonymous / PerkinElmer: "ATPlite: Luminescent ATP Detection System" PerkinElmer Internet Article 31 December 2002 (2002-12-31), pages 1-17, XP002589229 Retrieved from the Internet: URL:http://www.blossombio.com.tw/PDF/Produ cts/ATPlite.pdf [retrieved on 2010-06-28]
- Anonymous / PerkinElmer: "ATPlite 1step: Single Addition Luminescent ATP Detection System" PerkinElmer Internet Article 31 December 2003 (2003-12-31), pages 1-22, XP002589230 Retrieved from the Internet: URL:http://las.perkinelmer.com/content/Man uals/MAN_ATPLite1step.pdf [retrieved on 2010-06-28]
- MULIAWAN SYLVIA Y ET AL: "Inhibitory potential of Quercus lusitanica extract on dengue virus type 2 replication", SOUTHEAST ASIAN JOURNAL OF TROPICAL MEDICINE AND PUBLIC HEALTH, PROJECT OD SEAMEO, BANGKOK, vol. 37, no. Suppl. 3, 1 January 2006 (2006-01-01), pages 132-135, XP009162242, ISSN: 0125-1562
- NOAH ET AL: "A cell-based luminescence assay is effective for high-throughput screening of potential influenza antivirals", ANTIVIRAL RESEARCH, ELSEVIER BV, NL, vol. 73, no. 1, 14 January 2007 (2007-01-14), pages 50-59, XP005830364, ISSN: 0166-3542, DOI: 10.1016/J.ANTIVIRAL.2006.07.006
- GONG E ET AL: "Development of robust antiviral assays for profiling compounds against a panel of positive-strand RNA viruses using ATP/luminescence readout", JOURNAL OF VIROLOGICAL METHODS, ELSEVIER BV, NL, vol. 151, no. 1, 1 July 2008 (2008-07-01), pages 121-125, XP022708503, ISSN: 0166-0934, DOI: 10.1016/J.JVIROMET.2008.03.012 [retrieved on 2008-04-22]

## Description

The present invention relates to a method for identifying inhibitors against dengue virus subtypes 1, 2, 3 or 4 and its use in a high throughput mode.

The family Flaviviridae includes approximately 60 enveloped, positive-strand RNA viruses, most of which are transmitted by an insect vector. Many members of this family cause significant public health problems in different regions of the world. The genomes of all flaviviruses sequenced thus far have the same gene order: 5'-C-preM-E-NS1-NS2A-NS2B-NS3-NS4A-NS4B-NS5-3' in which the first three genes code for the structural proteins the capsid (C), the precursor to the membrane protein (prM) and the envelope protein (E).

Dengue is a mosquito-borne viral disease which occurs in tropical and subtropical regions throughout the world. Dengue is characterized by fever, rash, severe headache and joint pain. Its mortality rate is low. However, over the past few decades, a more severe form of dengue, characterized by hemorrhage and shock (dengue hemorrhagic fever/dengue shock syndrome; DHF/DSS) has been observed with increasing frequency in children and young adults. DHF/DSS occurs most often during dengue virus infection in individuals previously infected with another dengue virus serotype. This has led to the suggestion that immune enhancement of viral replication plays a role in the pathogenesis of the more severe form of disease.

Dengue epidemics are a major public health problem in many tropical and subtropical areas where the vector mosquito species are abundant. Control of dengue fever and DHF/DSS is a major concern of public health. Consequently, the WHO has designated the dengue viruses as a high priority target for accelerated research and vaccine development. Despite 40 years of intensive research, safe and effective vaccines for dengue virus disease are not available.

Soon after their isolation in 1944, dengue viruses were passaged repeatedly in mouse brain, resulting in the selection of mouse neurovirulent mutants. Interestingly, studies performed in volunteers showed that mouse brain-adapted neurovirulent mutants of three strains of type 1 or type 2 dengue viruses were attenuated, but still immunogenic for humans. However, the mutants were not developed further as candidate vaccine strains because of concern for mouse brain antigens in the vaccine preparations. Since that time, virus mutants that: (i) exhibited the small plaque size phenotype, and/or (ii) were temperature sensitive, and/or (iii) were adapted to cell cultures derived from an unnatural host (i.e., host range mutants), have been selected and evaluated as candidates for inclusion in a live attenuated virus vaccine. However, despite 25 years of such efforts, safe, effective dengue vaccines are still not available for general use. Inactivated whole dengue virus vaccines have been shown to be insufficiently immunogenic. Live virus vaccines attenuated by serial passage in cell culture have suffered from genetic instability under attenuation or poor immunogenicity.

The four serotypes of dengue viruses (type 1 to type 4) are distinguishable by plaque reduction neutralization using serotype-specific monoclonal antibodies and by less specific tests using polyclonal sera. The existence of serotypes was first discovered during early studies in human volunteers, which showed that infection with one dengue serotype induced durable homotypic immunity, whereas heterotypic immunity lasted only 3 to 5 months. An effective dengue vaccine that contains all four serotypes in order to induce broad immunity to dengue viruses in general would help to preclude the occurrence of DHF/DSS.

The complete nucleotide sequences have been determined for all four dengue virus serotypes (Mackow, E. et al. (1987) Virology 159:217-228; Zhao, B. et al. (1986) Virology 155:77-88; Osatomi, K. & Sumiyoshi, H. (1990) Virology 176:643-647; Irie, A. et al. (1989) Gene 75:197-211; Mason, P. W. et al. (1987) Virology 161:262-267; Hahn, Y. S. et al. (1988) Virology 162:167-180). The results of these studies indicate that the four dengue virus serotypes share a common genome organization. The genome of the dengue type 4 Caribbean strain 814669 was found to contain 10646 nucleotides (Mackow, E. et al. (1987) Virology 159:217-228; Zhao, B. et al. (1986) Virology 155:77-88). The first 101 nucleotides at the 5' end and the last 384 at the 3' end are non-coding regions. The remaining sequence codes for a 3386 amino-acid polyprotein which includes the three structural proteins, namely, capsid (C), premembrane (prM), and envelope (E), at its N-terminus, followed by seven non-structural proteins in the order, provided above, that is consistent with all Flavivirus genomes identified thus far. The polyprotein is processed to generate 11 or more viral proteins by cell signal peptidase(s) and by viral proteases (Markoff, L. (1989) J. Virol, 63:3345-3352; Falgout, B. et al. (1989) J. Virol, 63:1852-1860; Falgout, B. et al. (1991) J. Virol. 65:2467-2476; Hori, H. & Lai, C. J. (1990) J. Virol. 64:4573-4577).

Since lacking a vaccine to combat dengue virus and its further spread and infection throughout the world population, a high medical need exists to have small chemical molecules, such as chemical compounds, available which could inhibit dengue virus serotypes 1-4.

In order to be able to find those small chemical molecules a screening effort is definitely needed to test several thousands and millions of chemical molecules present in a compound library for their potential application in inhibiting dengue virus replication and expression.

MULIAWAN SYLVIA Y ET AL: "Inhibitory potential of Quercus lusitanica extract on dengue virus type 2 replication", SOUTHEAST ASIAN JOURNAL OF TROPICAL MEDICINE AND PUBLIC HEALTH, vol. 37, Suppl. 3, January 2006, p.132-135, disclose an assay involving C6/36 cells. The inhibitory effect was determined by 2D gel electrophoresis.

NOAH ET AL: "A cell-based luminescence assay is effective for high-throughput screening of potential influenza antivirals", ANTIVIRAL RESEARCH, vol. 73, no. 1, Epub 28.07.2006, p.50-59, disclose an assay in which MDCK cells were used.

GONG E ET AL: "Development of robust antiviral assays for profiling compounds against a panel of positive-strand RNA viruses using ATP/luminescence readout", JOURNAL OF VIROLOGICAL METHODS, vol. 151, no. 1, Epub 22.04.2008, p.121-125, disclose cell-based assays involving Vero and Vero E6 cells.

A screening effort can only be accomplished with a good, fast and reliable high throughput screening assay wherein numerous of chemical compounds can be tested and analyzed in a high speed module.

The current invention relates to a method for identifying inhibitors against dengue viruses serotypes 1, 2, 3 or 4 by screening chemical compounds or chemical compound libraries comprising:
a) bringing about 1000 to 2000 Vero cells or Huh 7.5 cells into contact with a chemical compound,
b) adding dengue virus to said cells and chemical compound of step a),
c) incubating said cells, chemical compound and virus of step b) at 37°C until viral cytopathic effect (CPE) in the virus control reaches about 100%,
d) adding luciferase enzyme and luciferase substrate to the cells, compound, virus,
and measuring thereafter the luminescence and calculate the EC₅₀ value which is a measure for the inhibitory activity of the compound against dengue virus.

In the above method when dengue virus subtype 2 is used and tested for, the preferred amount of Vero cells is about 1500, while when dengue virus subtypes 1, 3 or 4 are used and tested for, the preferred cells are the so-called Huh 7.5 cells.

The MOI (Multiplicity of Infection) in the method when dengue virus subtype 2 is used and tested for is 0.1, 0.5 or 1.0, while preferably an MOI is used of 0.1. The MOI (Multiplicity of Infection) in the method when dengue virus subtype 1, 3 or 4 is used and tested for, falls in the range of 1.0 and 10.0, but preferably is 1.0 or 5.0.

In the method according to the invention the incubation of the cells (Vero or Huh 7.5) together with a chemical compound and the dengue virus subtype tested for, occurs till a 100% cytopathic effect (CPE) has been obtained in the virus control. The timing to obtain such CPE could run for 5 or 6 days at 37°C incubation.

The preferred substrate for the luciferase enzyme is D-Luciferin, while the method according to the invention can easily be adapted and used in a high throughput mode to test numerous amounts of compounds present in a chemical library.

In the present invention, the term "inhibitor" is used to refer to any chemical entity, such as chemical compound, small molecule, peptide, protein and the like, which inhibit the growth, replication and/or proliferation of dengue virus subtype 1, 2 , 3 or 4.

The term "Vero cells" and its respective cell line refer to the cell line which is derived from kidney epithelial cells of the African Green Monkey. The cell line was established in 1962 by Japanese scientists. (see Yasumura Y, Kawakita M (1963). "The research_for the SV40 by means of tissue culture technique". Nippon Rinsho 21 (6): 1201-1219.)

The term "Huh 7.5 cells" refers to those cells as a subline derived from Huh-7 hepatoma cells (Blight K J, McKeating J A, Rice C M. J Virol. 2002;76:13001-13014.)

The ATPLite™ system (Perkin-Elmer and WO 00/18253) is an **A**denosine **T**ri**P**hosphate (ATP) monitoring system based on firefly (*Photinus pyralis*) luciferase. This luminescence assay is an alternative to colorimetric, fluorometric and radioisotopic assays for the quantitative evaluation of proliferation and cytotoxicity of cultured mammalian cells. ATP monitoring can be used to assess the cytocidal, cytostatic and proliferative effects of a wide range of drugs, biological response modifiers and biological compounds. ATPLite is a true homogeneous high sensitivity ATP monitoring 1-step addition assay kit for the quantification of viable cells. The kit can be used for continuous process systems such as in-line systems in high throughput environments. These in-line systems do not require a long signal half-life since the time between addition of the reagent and reading the resulting luminescence is relatively short (minutes). The decrease of the luminescent light-output is approximately 15% after 30 minutes. This decrease is independent of cell numbers but may differ between cell type and medium. The maximum cell number that can be applied has been determined to be 50,000 cells per well for 96-well and 12,500 cells per well for 384-well micro plates. Because the kit needs no stabilization of the luminescence signal, high throughput is preserved.

ATP is a marker for cell viability because it is present in all metabolically active cells and the concentration declines very rapidly when the cells undergo necrosis or apoptosis. The ATPlite 1 step assay system is based on the production of light caused by the reaction of ATP with added luciferase and D-luciferin.

This is illustrated in the following reaction scheme:

ATP + D-Luciferin + O₂ + (Mg²⁺ + Luciferase enyme) → Oxyluciferin + + AMP + PPi + CO2 + Light

The emitted light is proportional to the ATP concentration within certain limits.

### Materials and methods

### 1. Cells and viruses

Vero cells (African green monkey kidney cells) were purchased from the European Collection of Cell Cultures (ECACC) and cultured in Eagle's Minimum Essential Medium (MEM; Invitrogen) supplemented with 10% fetal calf serum (FCS; Cambrex, Belgium), 2 mM L-glutamine, and 0.04% gentamycin (50 mg/ml). For drug treatment, the identical medium supplemented with 2% FCS was used.

All dengue viruses were purchased from the European Collection of Cell Cultures (ECACC).

### 2. Virus stocks and titrations

Dengue virus stocks were prepared using the Vero cells and titrated by measuring the 50% tissue culture infectious dose (TCID₅₀), the virus stock dilution that produced CPE in 50% of the cells at endpoint.

### 3. Antiviral assays (dengue serotype 2)

15 µl of Vero cells (1500 cells/well) were added to a 384-well white plate containing 10 µl of four-fold serially diluted testing compound in cell culture medium with 2% FCS. 15 µl of virus stock was then added to each well at a MOI of 0.1. Cell controls received only cells and medium, while virus controls received virus but no test compound. Plates were incubated at 37°C until the viral CPE in the virus control wells reached ~100% (5-6 days). ATPLite (Perkin Elmer) was added to all wells according to the supplier's instructions. Briefly, 40 µl of the reconstituted lyophilized substrate solution was added to each well. The plate was shaken at 700 rpm for 2 minutes and the luminescence was measured using a Viewlux apparatus (Perkin Elmer) by taking a 0.1-0.5-second integrated reading of each test plate. The results were expressed as EC₅₀ values defined as the concentration of compound achieving 50% inhibition of the virus-reduced luminescence signals as compared with the uninfected cell control. The signal-to-noise ratio of an assay is the ratio between the mean luminescent signals of the cell controls and the virus controls. The dynamic range is defined as the ratio between the signals at the last (maximal signal) and first point in the linear range of the dose-response curve.

### 4. Cytotoxicity assay

To test for cytotoxicity, cells were incubated with serial compound dilutions as described above but in the absence of virus. The 50% cytotoxic concentration (CC₅₀) was determined by comparing the luminescent signal of compound treated wells with cell control wells.

### 5. Assay reproducibility

Intra-assay reproducibility was measured by performing each experiment in three identical plates with each concentration of drug in triplicate. Inter-assay reproducibility was measured by performing three independent experiments at different times under the same experimental conditions.

**Table: the EC₅₀ values of three (3) selected compounds**

| | EC₅₀ (µM) | | | | | | |
|---|---|---|---|---|---|---|---|
| | Exp. # 1 | Exp. # 2 | Exp. # 3 | Exp. # 4 | Exp. # 5 | Exp. # 6 | Exp. # 7 |
| Ribavirin | 51.99 | 53.80 | 43.76 | 42.38 | 47.71 | 68.98 | 67.47 |
| 2'-C-methylcytidine | 13.49 | 14.64 | 10.28 | 10.43 | 6.73 | 14.03 | 17.41 |
| 6-Azauridine | 5.52 | 4.85 | 3.74 | 4.26 | 4.60 | 3.81 | 5.14 |

The above demonstrates that the method according to the invention can be used to test for the selection of compounds which inhibit dengue virus.

### 6. Antiviral assays (dengue serotypes 1, 3, and 4)

The antiviral assays for dengue serotypes 1, 3, and 4 are similar to that of dengue serotype 2 except Huh7.5 cells (1000 cells/well) were used. The virus input for dengue serotype 1 (strain TC974) and serotype 4 (strain H241) is MOI of 5, for dengue serotype 3 (strain H87) is MOI of 1. The incubation time is 5 days.

### 7. Comparison with dye uptake assay:

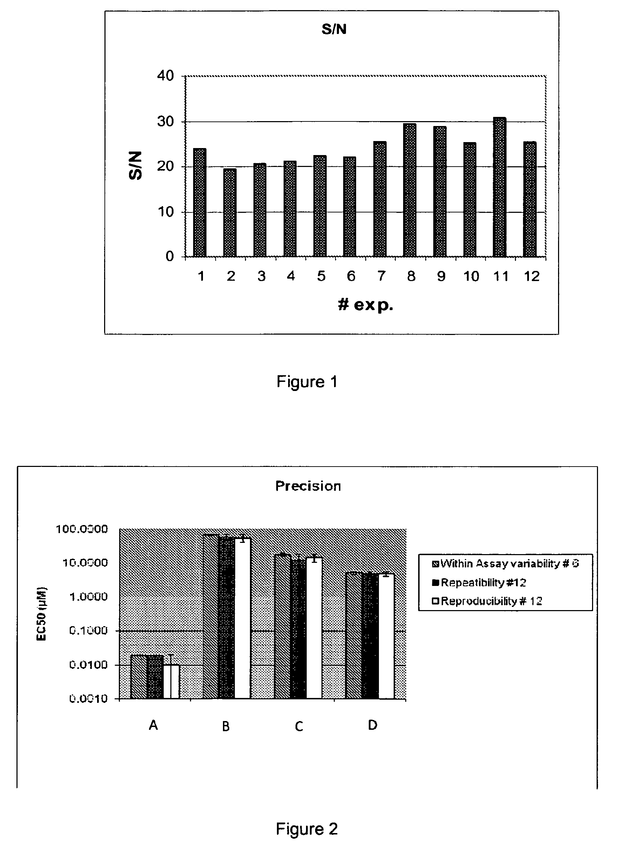

Cytopathic effect inhibition assays usually employ dye uptake readouts, e.g. neutral red and MTT (3-[4,5-dimethylthiazol-2-yl]-2,5-diphenyl tetrazolium bromide). The major drawbacks of such colorimetric readouts are low throughput, low dynamic range, and a low signal-to-noise ratio. For dengue virus, although 100% cytopathic effect was evident in infected cells, the presence of cell debris causes a high background, resulting in an extremely low signal-to-noise ratio and dynamic range. This renders the dye uptake assay unsuitable for dengue CPE inhibition assay. To overcome these disadvantages, an alternative assay using the ATP/luminescence readout was developed and validated. In comparison with dye uptake based assays, assays with ATP/luminescence based readout give unexpectedly a larger dynamic range and better signal-to-noise ratio (20-30 folds, see Figure 1). Further advantages of such assays are their high throughput and homogenous nature (mix and measure). As screening programs based on traditional CPE readouts are often hampered by problematic signal-to-noise ratios and low throughput, changing the end-point to a luminescent readout may has a significant impact on drug discovery.

### 8. The accuracy of the assay:

The table listed the EC₅₀s of the reference compounds and the number of measurements.

| **Compound** | **EC₅₀ (µM)** | **# data** |
|---|---|---|
| Ribavirin | 52.97 ± 10.21 | 53 |
| 2'-C-methylcytidine | 12.62 ± 3.32 | 54 |
| 6-Azauridine | 4.53 ± 0.83 | 42 |
| Interferon-α | 861.2 IU/ml ± 427.11 | 30 |

The robustness of the assay was determined by up to 24 measurements with variable operators, experiments, plate productions and virus stocks.

### 9. Library screening:

Using this HTS assay, a total of 150,000 compounds from different libraries were screened. The hit rate is ~0.83% (SI>4).

### 10. Precision of the assay:

The precision of the assay is determined by measuring the variability of the EC₅₀ values of the reference compounds A, B, C and D and shown in Figure 2.
- Within assay variability
- Repeatibility (within-day variability)
- Reproducibility (between-day variability)

## Claims

1. Method for identifying inhibitors against dengue viruses serotypes 1, 2, 3 or 4 by screening chemical compounds or chemical compound libraries comprising:
a) bringing about 1000 to 2000 Vero cells or Huh 7.5 cells into contact with a chemical compound,
b) adding dengue virus to said cells and chemical compound of step a),
c) incubating said cells, chemical compound and virus of step b) at 37°C until viral cytopathic effect (CPE) in the virus control reaches about 100%,
d) adding luciferase enzyme and luciferase substrate to the cells, compound, virus,
e) measuring the luminescence and calculate the EC₅₀ value which is a measure for the inhibitory activity of the compound against dengue virus.

2. Method according to claim 1 wherein the dengue virus is Dengue Virus subtype 2 and the cells are about 1500 Vero cells.

3. Method according to claim 1 wherein the dengue virus is Dengue Virus subtype 1, 3 or 4 and the cells are Huh 7.5 cells.

4. Method according to claim 1 or 2 wherein the dengue virus has a multiplicity of infection (MOI) of 0.1, 0.5 or 1.0, preferably 0.1.

5. Method according to claim 1 or 3 wherein the dengue virus has a multiplicity of infection (MOI) between 1.0 and 10.0, preferably 1.0 or 5.0.

6. Method according to claim 1-5 wherein step c) is about 5 or 6 days.

7. Method according to any of the claims 1-6 wherein the luciferase substrate is D-Luciferin.

8. Method according to any of the claims 1-7 wherein the method is performed in a high throughput mode.

## Patentansprüche

1. Verfahren zur Identifizierung von Hemmern gegen Dengue-Virus-Serotypen 1, 2, 3 oder 4 mittels Screening von chemischen Verbindungen oder Bibliotheken chemischer Verbindungen, wobei man:
a) etwa 1000 bis 2000 Vero-Zellen oder Huh 7.5-Zellen mit einer chemischen Verbindung in Kontakt bringt,
b) Dengue-Virus zu den Zellen und der chemischen Verbindung aus Schritt a) gibt,
c) die Zellen, die chemische Verbindung und das Virus aus Schritt b) bei 37°C inkubiert, bis etwa 100% Virus-CPE (Cytopathic Effect) bei der Viruskontrolle erreicht sind,
d) Luciferase-Enzym und Luciferase-Substrat zu den Zellen, der Verbindung, dem Virus gibt,
e) die Lumineszenz misst und den EC₅₀-Wert, bei dem es sich um ein Maß für die Hemmaktivität der Verbindung gegen Dengue-Virus handelt, berechnet.

2. Verfahren nach Anspruch 1, wobei es sich bei dem Dengue-Virus um Dengue-Virus-Subtyp 2 und bei den Zellen um etwa 1500 Vero-Zellen handelt.

3. Verfahren nach Anspruch 1, wobei es sich bei dem Dengue-Virus um Dengue-Virus-Subtyp 1, 3 oder 4 und bei den Zellen um Huh 7.5-Zellen handelt.

4. Verfahren nach Anspruch 1 oder 2, wobei das Dengue-Virus einen MOI-Wert (Multiplicity of Infection) von 0,1, 0,5 oder 1,0, vorzugsweise 0,1 aufweist.

5. Verfahren nach Anspruch 1 oder 3, wobei das Dengue-Virus einen MOI-Wert (Multiplicity of Infection) zwischen 1,0 und 10,0, vorzugsweise 1,0 oder 5,0 aufweist.

6. Verfahren nach Anspruch 1-5, wobei Schritt c) etwa 5 oder 6 Tage dauert.

7. Verfahren nach einem der Ansprüche 1-6, wobei es sich bei dem Luciferase-Substrat um D-Luciferin handelt.

8. Verfahren nach einem der Ansprüche 1-7, wobei das Verfahren im Hoher-Durchsatz-Modus durchgeführt wird.

## Revendications

1. Procédé destiné à identifier des inhibiteurs dirigés contre les sérotypes 1, 2, 3 ou 4 des virus de la dengue par un criblage de composés chimiques ou de banques de composés chimiques, comprenant les étapes consistant à :
a) amener environ 1 000 à 2 000 cellules Vero ou cellules Huh 7.5 à être en contact avec un composé chimique,
b) ajouter un virus de la dengue auxdites cellules et au dit composé chimique de l'étape a),
c) incuber lesdites cellules, ledit composé chimique et ledit virus de l'étape b) à 37°C jusqu'à ce que l'effet cytopathogène viral (CPE) dans le témoin viral atteigne environ 100%,
d) ajouter une enzyme luciférase ainsi qu'un substrat de la luciférase aux cellules, composé, virus,
e) mesurer la luminescence et calculer la valeur d'EC₅₀, qui est une mesure de l'activité d'inhibition du composé contre un virus de la dengue.

2. Procédé selon la revendication 1, dans lequel le virus de la dengue est le sous-type 2 du Virus de la Dengue et les cellules sont des cellules Vero au nombre de 1 500 environ.

3. Procédé selon la revendication 1, dans lequel le virus de la dengue est le sous-type 1, 3 ou 4 du Virus de la Dengue et les cellules sont des cellules Huh 7.5.

4. Procédé selon la revendication 1 ou la 2, dans lequel le virus de la dengue a une multiplicité d' infection (MOI) de 0,1, 0,5 ou 1,0, de préférence de 0,1.

5. Procédé selon la revendication 1 ou la 3, dans lequel le virus de la dengue a une multiplicité d'infection (MOI) comprise entre 1,0 et 10,0, de préférence de 1,0 ou de 5,0.

6. Procédé selon les revendications 1 à 5, dans lequel l'étape c) est de 5 ou 6 jours environ.

7. Procédé selon l'une quelconque des revendications 1 à 6, dans lequel le substrat de la luciférase est la D-Luciférine.

8. Procédé selon l'une quelconque des revendications 1 à 7, dans laquelle le procédé est exécuté dans un mode à haut débit.
